# EUROPEAN PATENT SPECIFICATION

(11) **EP 2 555 749 B1**
(45) Date of publication and mention of the grant of the patent: **26.10.2016**
(21) Application number: 11714895.7
(22) Date of filing: 05.04.2011
(51) Int. Cl.: A61K 31/382, A61K 31/4025, A61K 31/498, A61K 31/5578, A61K 9/00, A61K 31/5377

(54) **SUSTAINED-RELEASE RESERVOIR IMPLANTS FOR INTRACAMERAL DRUG DELIVERY**
RETARD-DEPOTIMPLANTATE FÜR INTRAKAMERALE WIRKSTOFFFREISETZUNG
IMPLANTS RÉSERVOIRS À LIBÉRATION PROLONGÉE POUR ADMINISTRATION DE MÉDICAMENT INTRACAMÉRULAIRE

(30) Priority: 06.04.2010 US 321422 P
(43) Date of publication of application: 13.02.2013
(73) Proprietor: ALLERGAN, INC., Irvine, CA 92612 (US)
(72) Inventor: SHI, Ruwen, Irvine California 92602 (US); HUGHES, Patrick M., Aliso Viejo California 92656 (US); BURKE, James A., Santa Ana California 92705 (US); ROBINSON, Michael R., Irvine California 92606 (US); LIU, Hui, Irvine California 92620 (US)
(74) Representative: Hoffmann Eitle
(86) International application number: PCT/US2011/031265
(87) International publication number: WO 2011/127064

(56) References cited:
- EP-B1- 2 114 531
- WO-A1-2011/075481
- US-A1- 2004 175 410
- US-A1- 2008 292 679
- US-A1- 2008 299 176

## Description

### CROSS REFERENCE TO RELATED APPLICATIONS

This Application is related to, and claims the benefit of, US provisional Patent Application 61/321,422, filed on April 6, 2010, in the names of Michael R. Robinson, et al.

### BACKGROUND OF THE INVENTION

The present invention relates to sustained release as defined by the claims for intraocular use, which implants are configured for primarily intracameral administration but also intrascleral, intracorneal, anterior vitreal administration to a patient suffering from an intraocular condition, said implant comprising a core of a drug, for treating said condition, surrounded by a polymer, which limits the rate of passage of the drug from the implant into the eye of said patient.

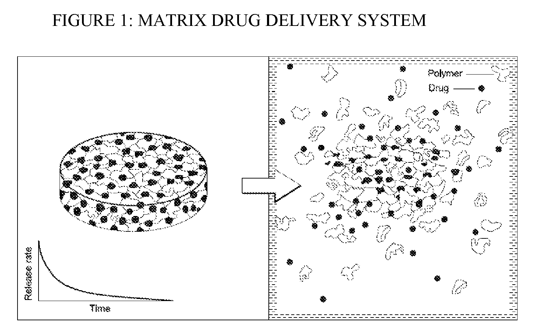

United States Patent Application Serial No. 12/411,250 describes sustained release matrix drug delivery systems, such as microspheres and implants, where the active pharmaceutical ingredient (API) is mixed homogenously with the polymer (See Figure 1). These matrix systems can be placed in the eye, such as in the anterior chamber (i.e. intracameral) or intravitreal, to release ocular anti-hypertensive drugs. The rate of drug released depends on the total surface area of the implant, the percentage of drug loaded, the water solubility of the API, and the speed of polymer degradation.

US 2004/175410 A1 discloses extended release implant devices comprising at least one carbonic anhydrase inhibitor for treatment of raised intra-ocular pressure. The drug is comprised in an inner drug-containing core within an outer polymeric layer, which is impermeable for the drug (e.g. ethylene/vinyl acetate polymer (EVA) and are adapted for insertion into the eye, e.g. the anterior or posterior chambers.

The present invention provides a sustained release as defined by the claims for intraocular use and, in particular, to treat elevated intraocular pressure, which implant is configured for intracameral or anterior vitreal administration to a patient with an ocular condition, e.g. elevated intraocular pressure (IOP), said implant comprising a core of an ocular drug. e.g. an antihypertensive agent, surrounded by a polymer, which limits the rate of passage of the drug or antihypertensive agent from the implant into the eye of said patient and said implant provides a linear rate of release of therapeutically effective amounts of said anti-hypertensive agent into the eye for a period of time of between approximately 14 days and 365 days.

In one aspect of the disclosure there is provided a reservoir implant suitable for releasing a hypotensive lipid, comprising a core made with a mixture of a hypotensive lipid and a biodegradable polymer, e.g. a polycaprolactone, or a nonbiodegradible polymer, e.g. a silicone elastomer, and/or an excipient, e.g. a surfactant such as a tri block copolymers of ethylene oxide and propylene oxide or an ethylene oxide adduct of a fatty acid or alcohol, extruded into thin filaments and coated with the rate limiting polymer, e.g. cellulose acetate, wherein said reservoir implant provides a linear release rate of hypotensive lipid over a period of 12 days or more.

In another aspect of the disclosure there is provided a reservoir implant suitable for releasing a hypotensive lipid, said implant comprising a core of said hypotensive lipid centrally located in a silicone tube having the ends closed by an impermeable ethylene vinyl acetate polymer, wherein the drug elutes from the sides of the silicone tube to provide a linear release over a period of 21 days or more.

### Brief Description of the Drawings

Figure 1 shows a matrix drug delivery system, as formed, and during the initial dissolving stage after placement in the eye.
Figure 2 shows the reservoir drug delivery system, as formed, and during the initial dissolving stage after placement in the eye.
Figure 3 shows the implant, described in Example 1, releasing the API, isopropyl 5-{3-[(2S)-1-{4-[(1S)-1-hydroxyhexyl] phenyl}-5-oxypyrrolidin-2-yl]propyl}thiophene-2-carboxylate at an in vitro release of 0.2ug/day.
Figure 4 shows a reservoir implant, as described in Example 1, releases a hypotensive lipid after being placed intracamerally in a dog to reduce the intraocular pressure approximately 30 to 45% below baseline for at least 5 weeks.
Figure 5 shows, a reservoir implant, as described in Example 1, releases a hypotensive lipid after being placed intravitreally in a dog to reduce the intraocular pressure to approximately a maximum of 15 to 20% below baseline over the initial 3 weeks.
Figure 6 shows the Implants described in Example 2, releasing the API, isopropyl 5-{3-[(2S)-1-{4-[(1S)-1-hydroxyhexyl] phenyl}-5-oxypyrrolidin-2-yl]propyl}thiophene-2-carboxylate at an in vitro release of 6.3ug/day and 9.3 ug/day.
Figure 7 shows, a reservoir implant, as described in example 2, releases a hypotensive lipid after being placed intracamerally in a dog to reduce the intraocular pressure to approximately 60% below baseline over a 2 week time frame.
Figure 8 shows an intracameral reservoir implant releasing an EP2 agonist as described in Example 2.
Figure 9 shows three reservoir implants, as described in example 2, release a hypotensive lipid after being placed sub-Tenon's in a dog to reduce the intraocular pressure to approximately a maximum of 18 to 20% below baseline over the initial 2 weeks.
Figure 10 shows sub-Tenon's reservoir implants (arrow) releasing an EP2 agonist as described in Example 2.
Figure 11 shows an implant, as described in Example 3, releases the API, isopropyl 5-{3-[(2S)-1-{4-[(1S)-1-hydroxyhexyl] phenyl}-5-oxypyrrolidin-2-yl]propyl}thiophene-2-carboxylate at an in vitro release rate of 46 ug/day and 66 ug/day.
Figure 12 describes certain implants of the invention comprising varying amounts of bimatoprost in the core surrounded by a polycaprolactone hollow tube of varying wall thicknesses.
Figure 13 shows the release rates of certain of the implants of Figure 12.
Figure 14 shows the release rates of certain of the implants of Figure 12.
Figure 15 shows the release rates of certain of the implants of Figure 12.

### Detailed Description

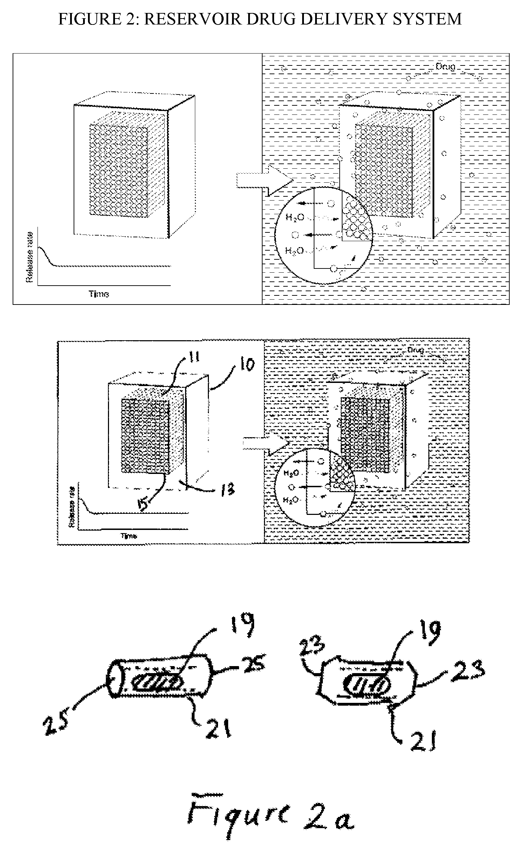

The present invention provides sustained release reservoir drug delivery systems for intracameral or intravitreal application as defined by the claims. Said reservoir systems comprise a drug reservoir surrounded by bioerodible or non-bioerodible polymers that control the drug release (See Figure 2).

As shown in Figure 2, the drug delivery system comprises an implant (10) configured for implantation in the anterior vitreal, space which implant comprises a core (11), which, in the embodiment shown in this figure, is fabricated as a bundle of individual fibers (15), said fibers comprising a drug for treating an ocular condition. Said drug may be combined with one or more excipients to form a mixture and the mixture extruded into fibers, which fibers are bundled to form a contiguous body to provide the core of the implant. The core is surrounded by a polymer (15) which is permeable to the drug and controls the passage of the drug from the core into the eye in which the implant is placed. In this embodiment, the rate limiting polymer completely surrounds the drug-containing core. However, the rate limiting polymer may not be the sole means of surrounding the core to isolate the core. As shown in Figure 2a the drug-containing core (19) may be centrally located in a tube (21) which tube may be a polymer which is permeable to the drug and controls the passage of the drug from the core into the eye in which the implant is placed. The tube heat sealed at one or both ends (23) or capped (25) at one or both ends with a drug impermeable polymer.

As further shown in Figure 2, water from the anterior chamber of the eye diffuses through the rate controlling polymers to the drug reservoir, dissolves the drug at the contact site of the drug reservoir, and the drug diffuses outwards from the polymer into the ocular tissues. The advantage of a reservoir drug delivery system over matrix drug delivery systems is that the reservoir delivers a smaller initial drug burst followed by a steady-state release rate that persists until the majority of the drug reservoir is depleted. The release rate is directly proportional to both the surface area of the implant, the diffusivity (i.e. the diffusion coefficient of the drug through the rate-limiting polymers), and indirectly proportional to the thickness of the surrounding polymers. The drug release from the reservoir implant can be tuned to the desired release rate by altering the surface area of drug diffusion, changing the polymer, and/or varying the thickness of the polymer coating. Another advantage of a reservoir implant is the ability to harbor large drug loads so that the implant can release for a minimum of 3 months and up to 5 years. In addition, the drug reservoirs and the rate-controlling polymer membranes can be fabricated using separate processes then assembled together to form implants. Mild fabrication process can be selected for making the drug reservoirs so that the activity and/or chemical integrity of the drugs or pharmaceutical agents in the reservoirs are protected from harsh conditions (high temperature and high shearing force) that may be needed for melt extrusion. Therefore, drug degradation is minimized. This is particularly useful for delivery of heat-labile drug compounds. The rate-controlling membranes can also shield the drugs in the reservoirs from enzymatic degradation.

The drug reservoirs can contain drug, only, or a mixture of drug and excipients. A variety of excipients can be incorporated in the formulations of the said drug reservoirs. These include, but are not limited to, surfactants, e.g. tri block copolymers of ethylene oxide and propylene oxide and ethylene oxide adducts of fatty acids or alcohols; anti-oxidants; pH modulating agents; bulking agents; osmotic agents; tonicity agents; disintegrating agents; binders, gliding agents; etc. For example, the said excipients can be selected from the following: Pluronic F68, Pluronic F127 (Polyoxamer 407), polysorbate 80, polysorbate 20, sodium dodecyl sulfate, hydroxypropyl-beta-cyclodextrin, poly(ethylene oxide), poly(ethylene glycol), polyvinylpyrrolidone, hydroxypropyl methylcellulose, carboxymethylcellulose, sodium phosphate, sodium chloride. The drug reservoirs can be fabricated using a various methods including compression, packing, and/or extrusion. The preferred surfactants are further described below:

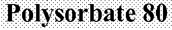

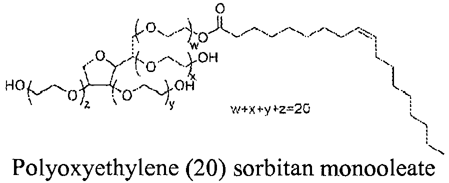

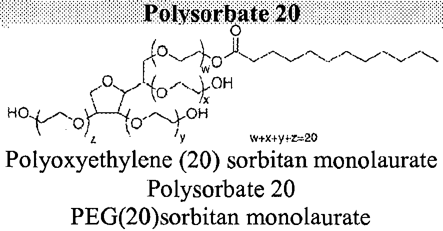

Poloxamer 407 is a hydrophilic non-ionic surfactant of the more general class of copolymers known as poloxamers. Poloxamer 407 is a triblock copolymer consisting of a central hydrophobic block of polypropylene glycol flanked by two hydrophilic blocks of polyethylene glycol. The approximate lengths of the two PEG blocks is 101 repeat units while the approximate length of the propylene glycol block is 56 repeat units. This particular compound is also known by the BASF trade name Pluronic F127.

Poloxamer 188, also known as Pluronic F68, is also a triblock copolymer with a similar chemical structure to Poloxamer 407 containing a center block of polypropylene glycol (PPG) flanked by a poly(ethylene glycol) (PEG) block on each side. The molecular weight of Poloxamer 188 is lower than Poloxamer 407.

The rate-controlling membranes surrounding the drug reservoirs are made of nondegradable polymers including, but not limited to, silicone elastomers, poly(ethylene-co-vinylacetate), polyurethane, or biodegradable polymers such as aliphatic polyesters. The membranes can be fabricated by solution casting, spray coating, or melt extrusion.

The implants can be fabricated in the following ways:
- Coating a pre-formed drug reservoir using conventional coating methods including dip coating, spray coating, etc.
- Inserting / filling a pre-formed drug reservoir into a pre-formed capsule made of said rate-controlling polymers.
- Co-extruding the drug reservoir formulation and the rate-controlling polymer.

In one embodiment of the invention, the rate-controlling membranes are made of degradable aliphatic polyesters such as, but not limited to, poly(ε-caprolactone), poly(D,L-lactide), poly(L-lactide), copolymers of lactones such as poly(D,L-lactide-co-glycolide), and mixtures of two or more of these polymers. The polymers can be melt-extruded or molded into capsules with one of the ends open. Drug reservoirs in their solid or liquid forms are then filled into the open-ended capsules and the open ends are subsequently sealed. The drug load can be released over time and the polymer structure bioerodes within ~6 to 12 months of drug release. The reservoir delivery systems can be also placed in the sub-Tenon's, subconjunctival, episcleral, intrascleral, suprachoroidal, intrachoroidal, and sub-retinal spaces.

Poly(ε-caprolactone) (PCL) is a biodegradable aliphatic polyester. It is usually prepared by ring-opening polymerization of ε-caprolactone using a catalyst such as stannous octoate. The chemical structure of PCL is as follows:

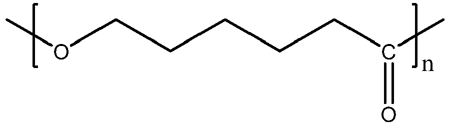

Examples of drugs that can be used with the reservoir delivery systems include the following:
- Hypotensive lipids (e.g. bimatoprost and compounds set forth in U.S. Pat. No. 5,352,708), and other prostaglandin analogues like latanoprost (Xalatan), bimatoprost (Lumigan), travoprost (Travatan), unoprostone, EP2/EP4 receptor agonists, and Asterand compounds. The prostaglandin analogues increase uveoscleral outflow of aqueous humor and bimatoprost also increases trabecular outflow.
- Topical beta-adrenergic receptor antagonists such as timolol, betaxolol, levobetaxolol, carteolol, levobunolol, and propranolol decrease aqueous humor production by the ciliary body.
- Alpha-adrenergic agonists such as brimonidine (Alphagan) and apraclonidine (iopidine) work by a dual mechanism, decreasing aqueous production and increasing uveoscleral outflow. Less-selective sympathomimetics like epinephrine and dipivefrin (Propine) increase outflow of aqueous humor through trabecular meshwork and possibly through uveoscleral outflow pathway, probably by a beta 2-agonist action.
- Miotic agents (parasympathomimetics) like pilocarpine work by contraction of the ciliary muscle, tightening the trabecular meshwork and allowing increased outflow of the aqueous humor.
- Carbonic anhydrase inhibitors like dorzolamide (Trusopt), brinzolamide (Azopt), acetazolamide (Diamox) lower secretion of aqueous humor by inhibiting carbonic anhydrase in the ciliary body.
- Other drugs that lower IOP can be used in the delivery system such as Rho-kinase inhibitors (e.g. INS 117548) designed to lower IOP by disrupting the actin cytoskeleton of the trabecular meshwork, Latrunculin B compound (e.g. INS 115644), PF-04217329, PF-03187207, AR-102, AL-6221, AL-3789, calcium channel blockers, vaptans (vasopressin-receptor antagonists), anecortave acetate and analogues, ethacrynic acid, and cannabinoids.

Combinations of ocular anti-hypertensives, such as a beta blocker and a prostaglandin analogue, can also be used in the delivery systems. These include Ganfort (bimatoprost/timolol), Extravan or Duotrav (travoprost/timolol), Xalcom (latanoprost/timolol, Combigan (brimonidine/timolol, and Cosopt (dorzolamide/timolol).

In combination with an IOP lowering drug, an agent that confers neuroprotection can also be placed in the delivery system and includes memantine and serotonergics [e.g., 5-HT.sub.2 agonists, such as S-(+)-1-(2-aminopropyl)-indazole-6-ol)].

Non-antihypertensive agents can also be used, such as anti-VEGF compounds to treat anterior or posterior segment neovascularization, or corticosteroids to treat uveitis, macular edema, and neovascular diseases.

The following examples are intended to illustrate the present invention.

### Example 1:

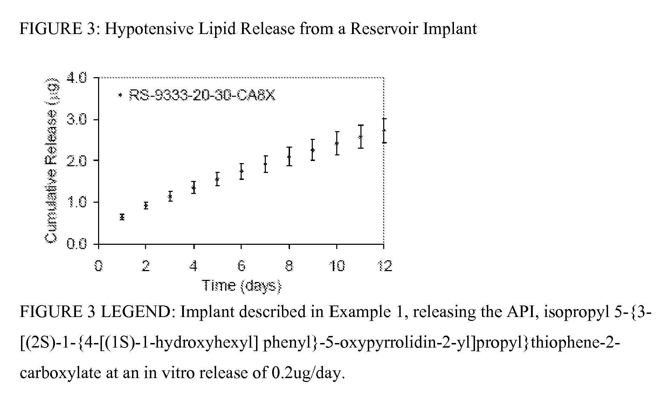

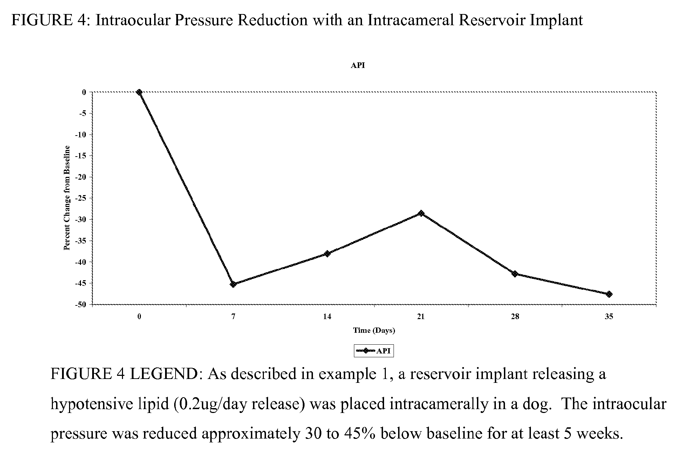

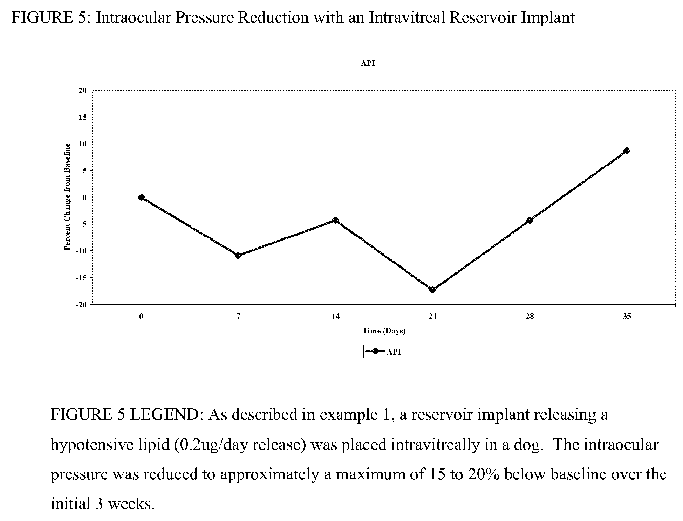

A reservoir implant releasing the hypotensive lipid, isopropyl 5-{3-[(2S)-1-{4-[(1S)-1-hydroxyhexyl] phenyl}-5-oxypyrrolidin-2-yl]propyl}thiophene-2-carboxylate ( an EP2 agonist), was formulated into a reservoir implant for intracameral and intravitreal application. The reservoir cores were made with a formulation comprising the hypotensive lipid, a poly(ε-caprolactone) and a poloxamer at a weight ratio of 2:6:2, e.g., Poloxamer 407 a triblock copolymer consisting of a central hydrophobic block of polypropylene glycol flanked by two hydrophilic blocks of polyethylene glycol, which was extruded into thin filaments and the cores were coated with cellulose acetate. The total drug loading was 200 ug and the in vitro release rates were 0.2ug/day (See Figure 3). The release rate was linear over a 12-day period. An intracameral injection of the implant was performed in a dog. The intraocular pressure was reduced approximately 30 to 45% below baseline for a minimum of 5 weeks (See Figure 4). A similar reservoir implant was placed intravitreally in a dog. The intraocular pressure was reduced to approximately a maximum of 15 to 20% below baseline over the initial 3 weeks (See Figure 5).

### Example 2

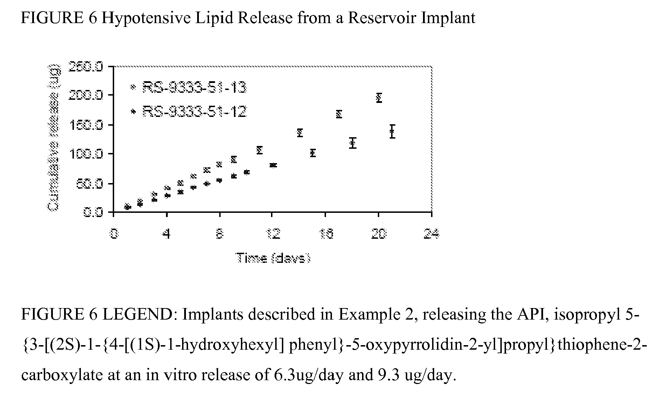

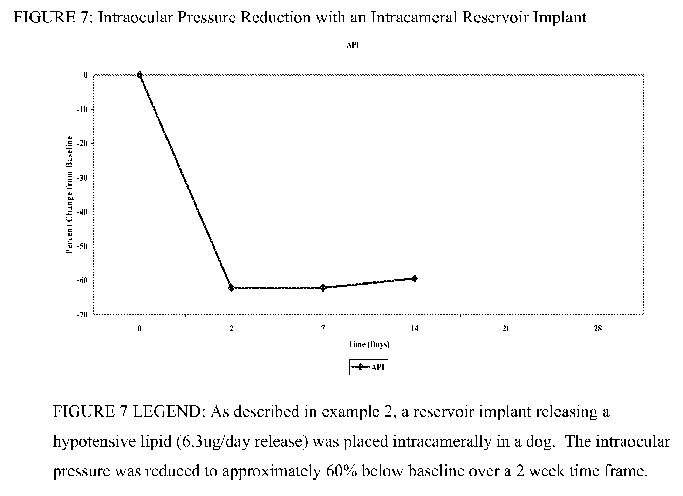

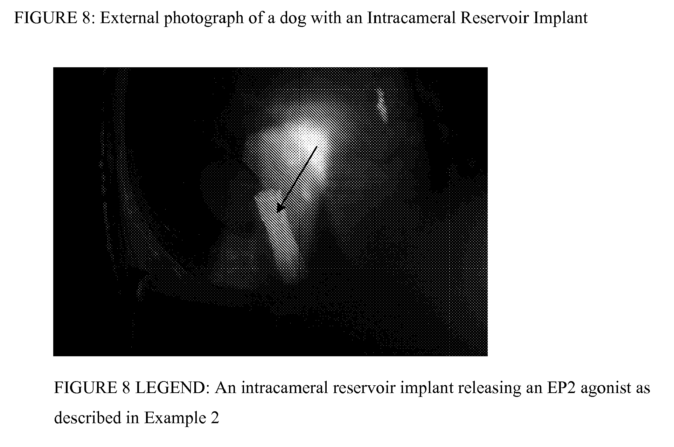

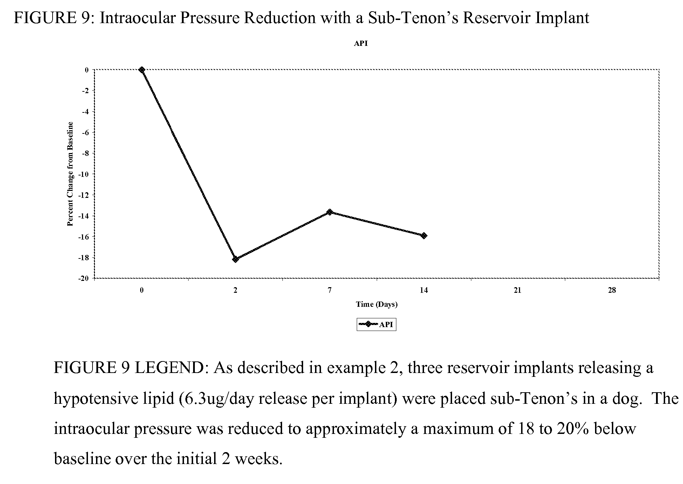

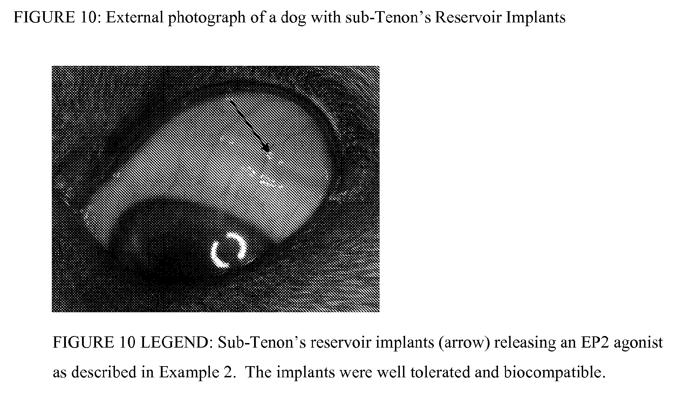

A reservoir implant releasing, isopropyl 5-{3-[(2S)-1-{4-[(1S)-1-hydroxyhexyl] phenyl}-5-oxypyrrolidin-2-yl]propyl}thiophene-2-carboxylate was formulated into a reservoir implant using a silicone tube, 1 mm in diameter. The drug reservoir was the API centrally located in tube and the ends were closed using ethylene vinyl acetate polymer. Implants with two effective lengths, 2 mm and 3 mm, were made. The drug loading was 563 µg in the 2 mm implants and 997 µg in the 3 mm implants. In vitro release rates of these implants were 6.3 µg/day and 9.3 ug/day for the 2 mm and 3 mm implants, respectively (See Figure 6). The drug elutes from the sides of the silicone tube with a linear release observed over a 21 day time period. Implantation of an implant was performed in the anterior chamber of a dog. An intracameral injection of an implant releasing at 6.3 ug/day was performed in a dog. There was a profound reduction in the intraocular pressure compared with the baseline (See Figure 7). The intracameral implant was well tolerated and biocompatible (See Figure 8). Three implants were placed in the sub-Tenon's space in a dog and the intraocular pressure was reduced to approximately a maximum of 18 to 20% below baseline over the initial 2 weeks (See Figure 9). The sub-Tenon's implants were well tolerated with no clinical signs of inflammation (See Figure 10).

### Example 3

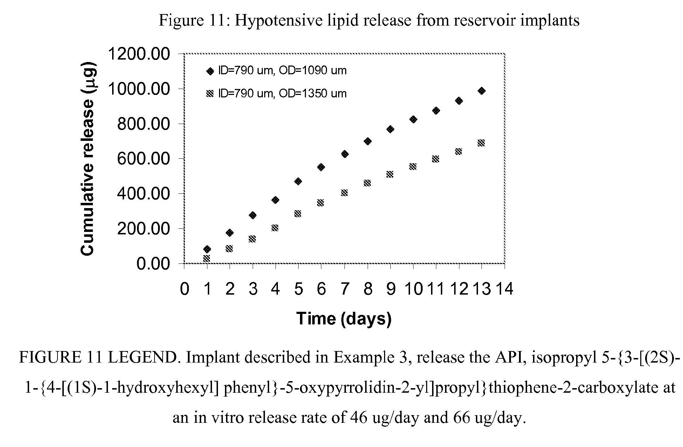

Poly(ε-caprolactone) (PCL) tubes with an inner diameter (ID) of 790 µm and outer diameters (OD) of 1090 µm and 1350 µm were cut into 6 mm in length. One of the open ends of the tubes was heat sealed, 1.5 mg of isopropyl 5-{3-[(2S)-1-{4-[(1S)-1-hydroxyhexyl] phenyl}-5-oxypyrrolidin-2-yl]propyl}thiophene-2-carboxylate, an EP2 agonist, was filled into each of the tubes using a syringe. The open end was then heat sealed to form a capsule-like implant. In vitro release profiles are shown in Figure 11. The drug release rate was 46 µg/day for the implants with the outer diameter of 1090 µm and 66 µg/day for the ones with the outer diameter of 1350 µm.

### Example 4

A sustained release implant comprising a core of an antihypertensive agent surrounded by a polymer, and configured for intracameral or anterior vitreal administration to a patient, is used to treat a patient with elevated intraocular pressure (IOP). The polymer utilized in said implant limits the rate of passage of the antihypertensive agent from the implant into the eye of the patient and provides a linear rate of release of therapeutically effective amounts of the anti-hypertensive into the eye for from 12 days to 365 days. The implant comprises a nonbiodegradable polymer, selected from the group consisting of silicone elastomers, poly(ethylene-co-vinylacetate)and polyurethane. or the implant comprises a biodegradable polymer, i.e., an aliphatic polyester.

The antihypertensive agent is selected from the group consisting of hypotensive lipids, i.e. bimatoprost, latanoprost, travoprost, unoprostone, EP2 receptor agonists EP2/EP4 receptor agonists; beta-adrenergic receptor antagonists, i.e. timolol, betaxolol, levobetaxolol, carteolol, levobunolol and propranolol; alpha-adrenergic agonists, i.e. brimonidine and apraclonidine; sympathomimetics, i.e. epinephrine and dipivefrin; miotic agents, i.e. pilocarpine; carbonic anhydrase inhibitors, i.e. dorzolamide, brinzolamide and acetazolamide; Rho-kinase inhibitors, i.e. Latrunculin B compound, PF-04217329, PF-03187207, AR-102, AL-6221, and AL-3789, calcium channel blockers, vasopressin-receptor antagonists, i.e. vaptans, anecortave acetate and analogues and ethacrynic acid and cannabinoids. Alternatively, the antihypertensive agent is a combination of ocular anti-hypertensives, and the combination is selected from the group consisting of bimatoprost/timolol, travoprost/timolol, latanoprost/timolol, brimonidine/timolol, and dorzolamide/timolol.

Alternatively, the implant is a reservoir implant releasing a drug for treating an ocular condition, suitable for intracameral and intravitreal application to treat an ocular condition and comprises a core made with a formulation comprising the drug, a polycaprolactone and a polyoxamer, which formulation is extruded into thin filaments, assembled into a bundle and coated with cellulose acetate, wherein the reservoir implant provides a linear release rate of the drug over a 12 day period.

In this example, the intraocular pressure is reduced approximately 30 to 45% below baseline for a minimum of 5 weeks, or the intraocular pressure is reduced to approximately a maximum of 15 to 20% below baseline over the initial 3 weeks. The total drug loading is 200 µg. The drug release rate is 0.2 µg/day.

Alternatively, a reservoir implant releasing a hypotensive lipid, the implant and suitable for intracameral and intravitreal application is used to treat an ocular condition. Said implant comprises a core of the hypotensive lipid centrally located in a silicone tube having the ends closed by an impermeable ethylene vinyl acetate polymer, wherein the drug elutes from the sides of the silicone tube to provide a linear release over a 21 day time period. The silicone tube has a diameter of 1 mm. The hypotensive lipid is an EP2 agonist. The intraocular pressure is reduced approximately 18 to 20% below baseline for a minimum of 2 weeks when placed in the sub-Tenon's space.

Alternatively, a reservoir implant releasing a hypotensive lipid, the implant and suitable for intracameral and intravitreal application is used to treat an ocular condition. Said implant comprises a core of the hypotensive lipid centrally located in a polycaprolactone tube having the ends heat sealed wherein the drug elutes from the sides of the silicone tube to provide a linear release observed over a 14 day time period. The tube has an inner diameter of 790 µm. The tube may has an outer diameter of 1090 µm and releases drug at a rate of 46 µg/day or the tube has an outer diameter of 1350 µm and releases drug at a rate of 66 µg/day.

Alternatively the implant comprises from about 10 to about 50 weight percent of the anti-hypertensive agent and from about 50 to about 90 weight percent of the polymer.

### Example 5

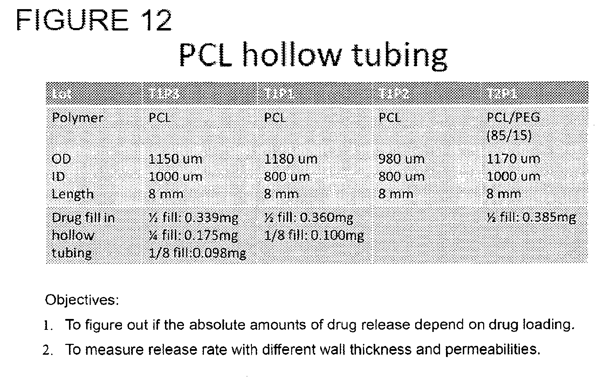

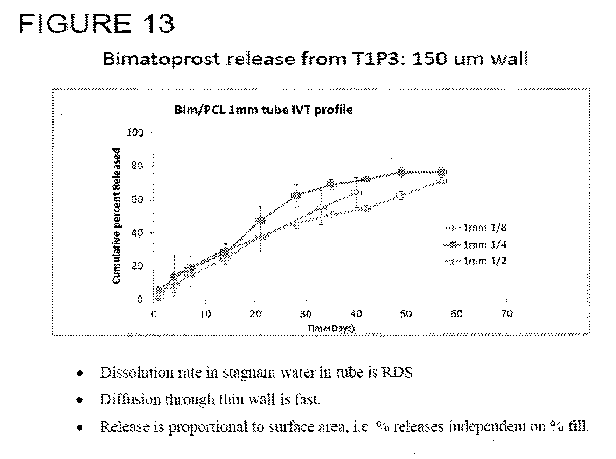

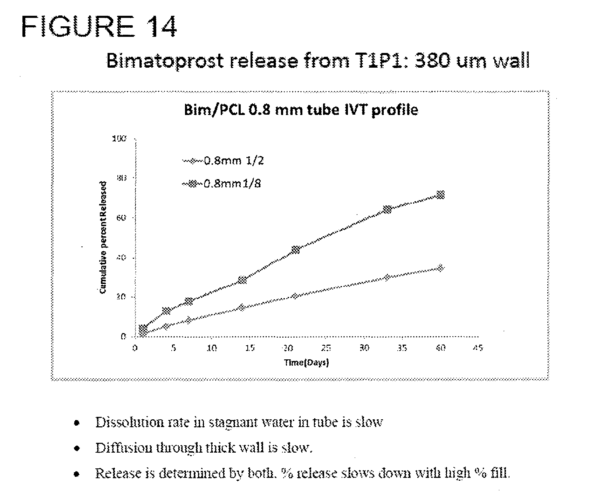

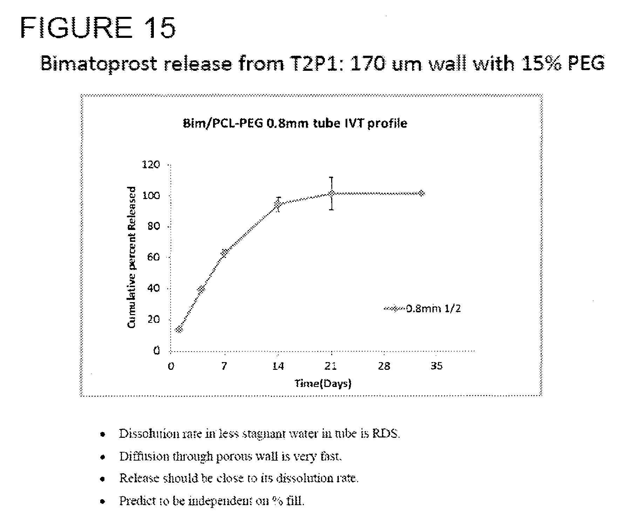

Poly(ε-caprolactone) (PCL) tubes with inner diameters (ID) of 800 µm and 1000 and an outer diameters (OD) of 980, 1150, 1170 and 1180 µm were cut into 8 mm lengths. One of the open ends of the tubes was heat sealed and varying amounts, i.e. from about 0.08 to 0.4 mg., of bimatoprost were filled into each of the tubes using a syringe. (See Figure 12.) The open end was then heat sealed to form a capsule-like implant. In-vitro release profiles are shown in Figures 13 through 15.

As a result of said experiment, the following conclusions were drawn:
PCL wall thickness affects permeability
With relatively thin walls, dissolution rate in the tubing is rate-determining step, as diffusion through the wall is fast and the release rate can be increased by increasing the filling.
With thick walls, both dissolution rate and wall thickness control release rate. Hydrophilic additives (e.g. PEG 3350) significantly increase release rates.

The present invention is not to be limited in scope by the exemplified embodiments, which are only intended as illustrations of specific aspects of the invention. It will be appreciated that the invention is not limited thereto.

## Claims

1. A sustained release implant for intraocular use to treat elevated intraocular pressure, configured for intracameral or anterior vitreal administration to a patient with elevated intraocular pressure (IOP), said implant comprising a core, which is fabricated as a bundle of individual fibres comprising an antihypertensive agent, said core is surrounded by a non-biodegradable polymer which limits the rate of passage of the antihypertensive agent from the implant into the eye of said patient, wherein:
the antihypertensive agent is selected from the group consisting of bimatoprost, latanoprost, travoprost, unoprostone, EP2/EP4 receptor agonists, timolol, betaxolol, levobetaxolol, carteolol, levobunolol, propranolol, brimonidine, apraclonidine, epinephrine, dipivefrin, pilocarpine, Latrunculin B compound, vaptans, anecortave acetate, ethacrynic acid, cannabinoids, brinzolamide, acetazolamide and EP2 receptor agonists; and
the implant provides a linear rate of release of therapeutically effective amounts of said anti-hypertensive into said eye for a period of time of between 12 days and 365 days.

2. The implant of Claim 1, wherein the non-biodegradable polymer is selected from the group consisting of silicone elastomers, poly(ethylene-co-vinylacetate) and polyurethane.

3. The implant of Claim 1, wherein the antihypertensive agent is selected from the group consisting of bimatoprost, latanoprost, travoprost, unoprostone, EP2 receptor agonists, and EP2/EP4 receptor agonists.

4. The implant of Claim 1 wherein antihypertensive agent is a combination of ocular anti-hypertensives.

5. The implant of Claim 4 wherein said combination is selected from the group consisting of bimatoprost/timolol, travoprost/timolol, latanoprost/timolol and brimonidine/timolol.

6. The implant of Claim 1, wherein said antihypertensive agent is an EP2 agonist.

7. An antihypertensive agent for use in treating elevated intraocular pressure, wherein the treatment comprises intracameral or anterior vitreal administration, to a patient with elevated intraocular pressure (IOP), of a sustained release implant according to Claim 1.

8. The antihypertensive agent for use according to Claim 7, wherein said implant comprises from about 10 to about 50 weight percent of said anti-hypertensive agent and from 50 to 90 weight percent of said polymer.

## Patentansprüche

1. Implantat mit verzögerter Freisetzung zur intraokularen Verwendung, um erhöhten Augeninnendruck zu behandeln, konfiguriert für intrakamerale oder anterior-vitreale Verabreichung an einen Patienten mit erhöhtem Augeninnendruck (IOP), wobei dieses Implantat einen Kern umfasst, der als ein Bündel einzelner Fasern, umfassend ein Antihypertonikum, gefertigt wurde, wobei dieser Kern von einem nicht biologisch abbaubaren Polymer umgeben ist, der die Passage-Geschwindigkeit des Antihypertonikums aus dem Implantat in das Auge dieses Patienten limitiert, wobei:
das Antihypertonikum aus der Gruppe, bestehend aus Bimatoprost, Latanoprost, Travoprost, Unoproston, EP2/EP4-Rezeptoragonisten, Timolol, Betaxolol, Levobetaxolol, Carteolol, Levobunolol, Propranolol, Brimonidin, Apraclonidin, Epinephrin, Dipivefrin, Pilocarpin, Latrunculin B-Verbindung, Vaptanen, Anecortave Acetat, Ethacrynsäure, Cannabinoiden, Brinzolamid, Acetazolamid und EP2-Rezeptoragonisten, ausgewählt ist; und
das Implantat eine lineare Freisetzungsgeschwindigkeit der therapeutisch wirksamen Mengen dieses Antihypertonikums in dieses Auge für eine Zeitspanne von zwischen 12 Tagen und 365 Tage bietet.

2. Implantat nach Anspruch 1, wobei das nicht biologisch abbaubare Polymer aus der Gruppe, bestehend aus Silikon-Elastomeren, Poly(ethylen-co-vinylacetat) und Polyurethan, ausgewählt ist.

3. Implantat nach Anspruch 1, wobei das Antihypertonikum aus der Gruppe, bestehend aus Bimatoprost, Latanoprost, Travoprost, Unoproston, EP2-Rezeptoragonisten und EP2/EP4-Rezeptoragonisten, ausgewählt ist.

4. Implantat nach Anspruch 1, wobei das Antihypertonikum eine Mischung von Augen-Antihypertonika ist.

5. Implantat nach Anspruch 4, wobei diese Mischung aus der Gruppe, bestehend aus Bimatoprost/Timolol, Travoprost/Timolol, Latanoprost/Timolol und Brimonidin/Timolol, ausgewählt ist.

6. Implantat nach Anspruch 1, wobei dieses Antihypertonikum ein EP2-Agonist ist.

7. Antihypertonikum zur Verwendung bei der Behandlung von erhöhtem Augeninnendruck, wobei die Behandlung intrakamerale oder anterior-vitreale Verabreichung, an einen Patienten mit erhöhtem Augeninnendruck (IOP), eines Implantats mit verzögerter Freisetzung nach Anspruch 1 umfasst.

8. Antihypertonikum zur Verwendung nach Anspruch 7, wobei dieses Implantat von etwa 10 bis etwa 50 Gewichtsprozent dieses Antihypertonikums und von 50 bis 90 Gewichtsprozent diese Polymers umfasst.

## Revendications

1. Implant à libération prolongée pour une utilisation intraoculaire pour le traitement d'une pression intraoculaire élevée, configuré pour une administration à l'intérieur de la chambre de l'oeil ou au niveau de la partie antérieur du corps vitré d'un patient ayant une pression intraoculaire élevée (PIO), ledit implant comprenant un noyau qui est fabriqué sous la forme d'un faisceau de fibres individuelles comprenant un agent antihypertenseur, ledit noyau étant entouré d'un polymère non biodégradable qui limite le taux de passage de l'agent antihypertenseur depuis l'implant vers l'oeil dudit patient, dans lequel,
l'agent antihypertenseur est choisi dans le groupe constitué par le bimatoprost, le latanoprost, le travoprost, l'unoprostone, des agonistes des récepteurs EP2/EP4, le timolol, le bétaxolol, le lévobétaxolol, le cartéolol, le lévobunolol, le propranolol, le brimonidine, l'apraclonidine, l'épinéphrine, le dipivéfrin, la pilocarpine, un composé de Latrunculine B, les vaptanes, l'acétate d'anécortave, l'acide éthacrynique, les cannabinoïdes, le brinzolamide, l'acétazolamide et les agonistes du récepteur EP2 ; et
l'implant fournit un taux linéaire de libération de quantités thérapeutiquement efficaces dudit antihypertenseur dans l'oeil pendant un laps de temps compris entre 12 jours et 365 jours.

2. Implant selon la revendication 1, dans lequel le polymère non biodégradable est choisi dans le groupe constitué par des élastomères de silicone, le poly(éthylène-co-acétate de vinyle) et le polyuréthane.

3. Implant selon la revendication 1, dans lequel l'agent antihypertenseur est choisi dans le groupe constitué par le bimatoprost, le latanoprost, le travoprost, l'unoprostone, d'agonistes des récepteurs EP2 et d'agonistes des récepteurs EP2/EP4.

4. Implant selon la revendication 1, dans lequel l'agent antihypertenseur est une combinaison d'antihypertenseurs oculaires.

5. Implant selon la revendication 4, dans lequel la combinaison est choisie dans le groupe constitué par le brimatoprost/timolol, le travoprost/timolol, le latanoprost/timolol et le brimonidine/timolol.

6. Implant selon la revendication 1, dans lequel l'agent antihypertenseur est un agoniste de EP2.

7. Agent antihypertenseur destiné à être utilisé dans le traitement de la pression intraoculaire élevée, dans lequel le traitement comprend une administration, à l'intérieur de la chambre de l'oeil ou au niveau de la partie antérieur du corps vitré, à un patient présentant une pression intraoculaire élevée (PIO), d'un implant à libération prolongée selon la revendication 1.

8. Agent antihypertenseur destiné à être utilisé selon la revendication 7, dans lequel ledit implant comprend d'environ 10 à environ 50 % en poids dudit agent antihypertenseur, et de 50 à 90 % en poids dudit polymère.
